# EUROPÄISCHE PATENTSCHRIFT

(11) **EP 4 327 075 B1**
(45) Veröffentlichungstag und Bekanntmachung des Hinweises auf die Patenterteilung: **10.06.2026**
(21) Anmeldenummer: 22723619.7
(22) Anmeldetag: 20.04.2022
(51) Int. Cl.: G01N 21/3563, G01N 21/359, G01N 21/86, G01N 33/34, G01N 21/84, G01N 21/27

(54) **VERFAHREN ZUR BESTIMMUNG DES QUANTITATIVEN VERHÄLTNISSES VON MELAMIN-FORMALDEHYD-HARZ UND HARNSTOFF-FORMALDEHYD-HARZ IN MINDESTENS EINER MIT EINER MISCHUNG AUS DIESEN HARZEN IMPRÄGNIERTEN PAPIERLAGE**
METHOD FOR DETERMINING THE QUANTITATIVE RATIO OF MELAMINE-FORMALDEHYDE RESIN AND UREA-FORMALDEHYDE RESIN IN AT LEAST ONE PAPER LAYER IMPREGNATED WITH A MIXTURE OF THESE RESINS
PROCÉDÉ DE DÉTERMINATION DU RAPPORT QUANTITATIF DE RÉSINE MÉLAMINE-FORMALDÉHYDE ET DE RÉSINE URÉE-FORMALDÉHYDE DANS AU MOINS UNE COUCHE DE PAPIER IMPRÉGNÉE D'UN MÉLANGE DESDITES RÉSINES

(30) Priorität: 22.04.2021 EP 21169864
(43) Veröffentlichungstag der Anmeldung: 28.02.2024
(73) Patentinhaber: Flooring Technologies Ltd., Kalkara SCM1001 (MT)
(72) Erfinder: ZHANG, Jinming, 10247 Berlin (DE); KALWA, Norbert, 32805 Horn-Bad Meinberg (DE)
(74) Vertreter: Maikowski & Ninnemann Patentanwälte Partnerschaft mbB
(86) Internationale Anmeldenummer: PCT/EP2022/060334
(87) Internationale Veröffentlichungsnummer: WO 2022/223568

(56) Entgegenhaltungen:
- EP-A1- 3 075 906
- US-A- 5 680 320
- US-A1- 2007 131 862
- HENRIQUES ANA ET AL: "Determination of resin and moisture content in melamine-formaldehyde paper using near infrared spectroscopy", JOURNAL OF NEAR INFRARED SPECTROSCOPY, vol. 25, no. 5, 1 October 2017 (2017-10-01), GB, pages 311 - 323, XP055843398, ISSN: 0967-0335, DOI: 10.1177/0967033517732122

## Beschreibung

Die vorliegende Erfindung betrifft ein Verfahren zur Bestimmung des quantitativen Verhältnisses von Melamin-Formaldehyd-Harz und Harnstoff-Formaldehyd-Harz in mindestens einer mit einer Mischung aus diesen Harzen imprägnierten Papierlage und eine Imprägnieranlage zur Durchführung dieses Verfahrens und die Verwendung der bestimmten Parameter zur Steuerung einer solchen Imprägnieranlage.

In der Holzwerkstoffindustrie werden in großem Stil imprägnierte Papiere zur Oberflächenveredelung von Holzwerkstoffplatten eingesetzt. Dies sind im wesentlichen Dekorpapiere, Overlays und Gegenzugpapiere. Diese imprägnierten Papiere werden in sogenannten Kurztaktpressen (KT-Pressen) bei hohem Druck und hohen Temperaturen auf Holzwerkstoffe aufgepresst. Diese beschichteten Platten werden dann in den verschiedensten Anwendungen genutzt (Möbel, Innenausbau, Laminatböden usw.).

Zur Imprägnierung der Papiere werden vor allem wässrige Melamin- und Harnstoffharze eingesetzt. Dabei finden die preisgünstigeren Harnstoffharze ihre Anwendung meist in der sogenannten Kernimprägnierung der Papiere, in Abmischung mit Melaminharzen, wohingegen reine Melaminharze meist in der Oberfläche der Papiere eingesetzt werden.

Bei der Herstellung der Imprägnate erfolgt der Auftrag des Harnstoffharzes mit einem Melaminanteil in einer ersten Imprägnierwanne, wo nach dem Eintauchen in die Imprägnierwanne ein Abstreifen des Harzes auf einen definierten Harzauftrag mit Hilfe von Rakeln oder Quetschwalzen erfolgt. Dann wird das Imprägnat in einem Schwebetrockner mit heißer Luft getrocknet und anschließend ein Deckstrich aus Melaminharz mit sich anschließender nochmaliger Trocknung aufgetragen wird. Beispielhaft sei auf die EP 3075906 B1 hingewiesen, in der eine Imprägnieranlage aus erster Imprägnierstation mit nachgeschalteter Trockenstation und einer zweiten Imprägnierstation mit nachgeschalteter Trockenstation beschrieben wird.

Im Imprägnierungsprozess ist eine Bestimmung des Verhältnisses der Auftragsmengen der Harze nach dem ersten Werk nur schwer möglich. Die Bestimmung kann nur durch Entnahme einer Probe aus dem laufenden Prozess erfolgen, wobei durch die hohe Geschwindigkeit der Anlage (60 - 100 m/min) und die Tatsache, dass es sich um feuchtes Papier handelt, die Gefahr von Papierabrissen besteht. Zudem kann die Probe nur aus den Randbereichen der Warenbahn entnommen werden.

Eine andere Möglichkeit wäre, zunächst nur ein Vor-/Kernimprägnierung vorzunehmen und dort zunächst die Parameter der Vorimprägnierung zu prüfen. Dies führt aber zu erheblichen Materialverlusten, da diese Imprägnate nicht verwendet werden können. Zudem müsste dieser Prozess bei jedem Wechsel auf sich stark unterscheidende Papiere wiederholt werden.

Zudem kann nur eine Aussage über die Gesamtharzmenge gemacht werden. Die Anteile sollten zwar über vorgegebene Rezepturen festgelegt sein, können aber wegen der verschiedensten Gründe von den Vorgaben abweichen (Fehlrezeptierungen, Dosier- und Mischprobleme).

Selbstverständlich können bei der Imprägnierung von Papieren auch durch Schwankungen bei den Rohstoffen (z. B. Harze mit unterschiedlichen Feststoffgehalten), durch die Produktionsparameter oder durch Produktionsfehler Produkte mit unklarem Mengenverhältnissen im Kern der Imprägnate entstehen. Es kann dazu führen, dass die Produktqualität von Charge zu Charge unterschiedlich sein könnte. Dies hat natürlich auch einen Einfluss auf die Kosten und die Qualität des Produktes, was bei dem Massenprodukt Imprägnat kritisch sein kann.

Die sich daraus ergebenen Nachteile sind eine erschwerte Fehleranalyse, steigende Kosten und eine schwierige Qualitätskontrolle.

Aus der EP 3 075 906 A1 ist ein Verfahren zur Bestimmung der Beharzung einer imprägnierten Papierlage bekannt, wobei die Beharzung der prozentuale Anteil von Harz am Papiergewicht ist. Als Harze kommen für eine erste Grund-Imprägnierung Harnstoffharz und Melaminharz zum Einsatz, für eine zweite Imprägnierung reines Melaminharz. Die Beharzung wird mittels NIR-Messköpfen bestimmt, welche an verschiedenen Stellen der Imprägnieranlage angeordnet sind. Die NIR-Spektren werden chemometrisch und unter Zuhilfenahme von Kalibrierdaten ausgewertet. Die ermittelte Beharzung wird zur Steuerung der Imprägnieranlage verwendet.

US 2007/131862 A1 beschreibt ein NIR-Spektroskopieverfahren zur Bestimmung der Zusammensetzung von Harz-Holz-Verbundwerkstoffen. Zur Bestimmung der Zusammensetzung des Harz-Holz-Verbundwerkstoffes wird ein Kalibrationsmodell eingesetzt, welches anhand von Referenzproben erstellt wurde. Mit dem Verfahren können verschiedene Harze gleichzeitig bestimmt werden.

US 5 680 320 A zeigt ein NIR-Verfahren zur Quantifizierung verschiedener Substanzen in imprägniertem Papier. Ausgehend von Referenzproben bekannter Zusammensetzung wird ebenfalls ein Kalibriermodell erstellt, welches mit den gemessenen Spektren verglichen wird. Zu den zu bestimmenden Substanzen gehören wet strength agents, welche die Nassfestigkeit von Papier erhöhen, wie zum Beispiel Harnstoffharz und Melaminharz.

Der Erfindung liegt daher die technische Aufgabe zu Grunde, ein Verfahren zu entwickeln, dass das Verhältnis zwischen Harnstoff- und Melaminharz schnell quantitativ bei den imprägnierten Papieren prüfen lässt. Das Verfahren sollte zur Überwachung der Produktion dienen. Die Prüfung sollte zerstörungsfrei erfolgen, sollte über die gesamte Warenbahnbreite möglich sein und kontinuierlich Daten liefern.

Diese Aufgabe wird durch ein Verfahren mit den Merkmalen des Anspruchs 1 gelöst.

Demnach wird ein Verfahren zur Bestimmung des quantitativen Verhältnisses von Melamin- und Harnstoff-Formaldehyd-Harz in mindestens einer mit einer Mischung aus diesen Harzen imprägnierten Papierlage bereitgestellt, wobei die Imprägnierung in einer Anlage zur Imprägnierung von mindestens einer durch die Anlage laufenden Papierlage durchgeführt wird. Das vorliegende Verfahren umfasst die folgenden Schritte:
- Imprägnieren von mehreren Papierlagen mit Harzmischungen mit jeweils unterschiedlichen quantitativ definierten Verhältnissen an Melamin-Formaldehyd-Harz und Harnstoff-Formaldehyd-Harz als Referenzproben;
- Aufnahme von mindestens einem NIR-Spektrum jeder der Referenzproben unter Verwendung von mindestens einem NIR-Messkopf in einem Wellenlängenbereich zwischen 500 nm und 2500 nm, bevorzugt zwischen 700 nm und 2000 nm, insbesondere bevorzugt zwischen 900 nm und 1700 nm, und besonders vorteilhaft zwischen 1450 nm und 1550 nm;
- Zuordnung der unterschiedlichen quantitativen Verhältnisse an Melamin-Formaldehyd-Harz und Harnstoff-Formaldehyd-Harz in den Referenzproben zu den aufgenommenen NIR-Spektren der genannten Referenzproben; und
- Erstellung eines Kalibriermodells für den Zusammenhang zwischen den spektralen Daten der NIR-Spektren und den dazugehörigen quantitativen Verhältnissen an Melamin- und Harnstoff-Formaldehyd-Harz der Referenzproben mittels einer multivariaten Datenanalyse;
- Imprägnieren von mindestens einer Papierlage mit einer Harzmischung aus Melamin- und Harnstoff-Formaldehyd-Harz;
- Aufnehmen von mindestens einem NIR-Spektrum der imprägnierten Papierlage unter Verwendung des mindestens einen NIR-Messkopfes in einem Wellenlängenbereich zwischen 500 nm und 2500 nm, bevorzugt zwischen 700 nm und 2000 nm, insbesondere bevorzugt zwischen 900 nm und 1700 nm und besonders vorteilhaft zwischen 1450 nm und 1550 nm; und
- Bestimmen des quantitativen Verhältnisses von Melamin- und Harnstoff-Formaldehyd-Harz in der imprägnierten Papierlage durch Vergleich des für die imprägnierte Papierlage aufgenommenen NIR-Spektrums mit dem erstellten Kalibriermodell, wobei die unter Einsatz des mindestens einen Messkopf ermittelten Daten zur Anlagensteuerung und Anlagenregelung verwendet werden.

Es erfolgt somit der Einsatz eines NIR-Messkopfes, der über die Aufnahme von spektralen Daten (Spektren) in nah infrarotem Bereich (950-1650 nm) eine Bestimmung der melaminharzimprägnierten Papiere, die gewisse oder ungewisse Mengen des Harnstoffharzes enthalten, zulässt. Die NIR-Strahlung wechselwirkt mit den organischen Funktionsgruppen, wie zum Beispiel O-H, C-H und N-H, die sowohl in Harn- als auch in Melaminharz vorhanden sind. Während der Wechselwirkung wird die NIR-Strahlung durch die gemessene Probe gestreut und reflektiert. Durch den Empfang der reflektierten NIR-Strahlung per eines NIR-Detektor wird ein NIR-Spektrum erzeugt. Dutzende NIR-Strahlung werden in einer Sekunde bei der Messung durchgeführt, sodass auch eine statische Absicherung der Werte gewährleistet wird. Das vorliegende Verfahren ermöglicht einen quantitativen Nachweis des Harnstoffharzes in melaminharzimprägnierten Papieren.

Harnstoff- und Melaminharz weisen sehr ähnliche NIR-Spektren auf, wobei der Harnstoffharzpeak in der Schulter vom Melaminharzpeak liegt. Durch die Überlagerung der verwendeten Harze kommt es zu einer Abschwächung des Harnstoffharzpeaks im Spektrum, wodurch die Analyse erschwert wird. Aus diesem Grund war es überraschend, dass ein Nachweis des Harnstoffharzes melaminharzimprägnierten Papieren überhaupt möglich war.

Zwar ist die Bestimmung der Konzentrationen von unterschiedlichen Harztypen, Wachs, Feuchtigkeit und andere Additive durch NIR Spektroskopie in Harz-Holz-Kompositen bekannt (siehe US 2007/0131862 A1), jedoch erfolgt die Bestimmung der Konzentrationen der genannten Komponenten an einer Mischung aus (vereinzelten) Holzpartikeln und Bindemittel vor dem Verpressen dieser Mischung zu entsprechenden Holz-Kompositen bzw. Holzprodukten. Im Gegensatz ermöglicht das vorliegende Verfahren die Bestimmung des quantitativen Verhältnisses von Melamin-Formaldehyd-Harz und Harnstoff-Formaldehyd-Harz in einer imprägnierten Papierlage nach einer Zwischentrocknung.

In einer Ausführungsform des vorliegenden Verfahrens erfolgt die Bestimmung des quantitativen Verhältnisses von Melamin-Formaldehyd-Harz und Harnstoff-Formaldehyd-Harz in einer imprägnierten Papierlage nach einem Trocknungsschritt, insbesondere einer Zwischentrocknung, wobei der Wassergehalt der Papierlage bevorzugterweise auf 10-20 Gew% reduziert ist.

In einer weiteren Ausführungsform des vorliegenden Verfahrens erfolgt die Bestimmung des quantitativen Verhältnisses von Melamin- und Harnstoff-Formaldehyd-Harz in mindestens einer mit dieser Harzmischung vorimprägnierten (bzw. kernimprägnierten) Papierlage.

In einer noch weiteren bevorzugten Ausführungsform des vorliegenden Verfahrens wird die Bestimmung des quantitativen Verhältnisses von Melamin- und Harnstoff-Formaldehyd-Harz in mindestens einer mit dieser Harzmischung vorimprägnierten (bzw. kernimprägnierten) Papierlage nach Verlassen einer (ersten) Imprägnierstation als Teil einer Anlage zur Imprägnierung von mindestens einer durch die Anlage laufenden Papierlage vorgenommen. Bevorzugt erfolgt im Anschluss an Vorimprägnierung der Papierlage in einer ersten Imprägnierstation eine Oberflächenimprägnierung der vorimprägnierten Papierlage in einer zweiten Imprägnierstation. Eine derartige Imprägnieranlage wird weiter unten noch im Detail beschrieben.

Die Bestimmung des quantitativen Verhältnisses von Melamin-Formaldehyd-Harz und Harnstoff-Formaldehyd-Harz erfolgt somit zwischen Vorimprägnierung und Oberflächenimprägnierung der durch die Imprägnieranlage laufenden Papierbahn, d.h. zwischen erster und zweiter Imprägnierstation.

Das vorliegende Verfahren erfordert lediglich eine einzelne NIR-Messung zur Bestimmung des quantitativen Verhältnisses von Melamin-Formaldehyd-Harz und Harnstoff-Formaldehyd-Harz. Vorteilhaft ist ebenfalls, dass die Vermessung an einer durchgängigen Papierlage durchgeführt wird, d.h. die Papierlage wird nicht zerstört.

Gemäß dem vorliegenden Verfahren werden zunächst Referenzproben der mit einer definierten Mischung aus Melamin- Formaldehyd-Harz und Harnstoff-Formaldehyd-Harz imprägnierten Papierlage bereitgestellt. Wesentlich ist, dass die Referenzprobe gleichartig zu der zu vermessenden Probe ist; d.h. insbesondere die Harzmischung der Referenzprobe weist die gleiche Zusammensetzung wie die zu vermessende Harzmischung auf. Die Gleichartigkeit von zu vermessender Probe und Referenzprobe ist insbesondere bei Verwendung von Harzmischungen mit Zusatzstoffen wie Flammschutzmitteln, Fasern, weiteren Additiven wesentlich.

Von diesen Referenzproben werden zumindest ein NIR-Spektrum in einem Wellenlängenbereich zwischen 500 nm und 2500 nm, bevorzugt zwischen 700 nm und 2000 nm, insbesondere bevorzugt zwischen 900 nm und 1700 nm aufgenommen.

Die quantitative Zusammensetzung der für die Referenzproben verwendeten Harzmischungen ist bekannt. So kann das quantitative Verhältnis von Melamin-Formaldehyd-Harz und Harnstoff-Formaldehyd-Harz in der zur Vorimprägnierung verwendeten Harzmischung zwischen 90 Gew% : 10 Gew% und 10 Gew% : 90 Gew%, bevorzugt zwischen 75 Gew% : 25 Gew% und 25 Gew%: 75 Gew%, insbesondere bevorzugt zwischen 55 Gew%: 45 Gew% und 45 Gew%: 55 Gew% betragen.

Auch liegt der Harzanteil in einer imprägnierten Papierlage bei > 100%. Wenn man das Papiergewicht als Basis nimmt, liegt der Harzauftrag üblicherweise bei ca. 100 - 140 %, wie z.B, bei Dekorimprägnaten. Bei spezielleren Imprägnaten kann der Harzanteil noch deutlich höher liegen wie z.B. bei Overlaypapierimprägnaten.

Die bekannte quantitative Zusammensetzung der Harzmischung wird dann den jeweils aufgenommen NIR-Spektren dieser Referenzproben zugeordnet wird, und es wird ein Kalibriermodell für den Zusammenhang zwischen den spektralen Daten der NIR-Spektren der Referenzproben und den dazugehörigen Parameterwerten mittels einer multivariaten Datenanalyse erstellt; d.h. zu jedem Parameterwert der Referenzprobe korrespondiert ein NIR-Spektrum der Referenzprobe. Die für die verschiedenen Parameter erstellten Kalibriermodelle werden in einem geeigneten Datenspeicher hinterlegt.

Anschließend wird mindestens eine Papierlage mit einer Harzmischung aus Melamin- und Harnstoff-Formaldehyd-Harz (vor)imprägniert und mindestens ein NIR-Spektrum der (vor)imprägnierten Papierlage aufgenommen. Das quantitative Verhältnis aus Melamin- und Harnstoff-Formaldehyd-Harz in der (vor)imprägnierten Papierlage kann dann durch Vergleich des für die Harzschicht aufgenommenen NIR-Spektrums mit dem erstellten Kalibriermodell bestimmt werden.

Ein Vergleich und die Interpretation der NIR-Spektren erfolgt sinnvollerweise über den gesamten aufgenommenen Spektralbereich. Dies wird vorteilhaft mit einer an sich bekannten multivariaten Datenanalyse (MDA) durchgeführt. Bei multivariaten Analysemethoden werden in an sich bekannter Weise typischerweise mehrere statistische Variablen zugleich untersucht. Hierzu wird bei diesen Methoden üblicherweise die in einem Datensatz enthaltene Zahl von Variablen reduziert, ohne gleichzeitig die darin enthaltene Information zu mindern.

Im vorliegenden Fall erfolgt die multivariate Datenanalyse über das Verfahren der Partial Least Squares Regression (partielle Regression der kleinsten Quadrate, PLS) wodurch ein geeignetes Kalibrationsmodell erstellt werden kann. Die Auswertung der gewonnenen Daten wird bevorzugt mit einer geeigneten Analysesoftware vorgenommen wie z.B. mit der Analysesoftware SIMCA-P der Firma Umetrics AB oder The Unscrambler der Firma CAMO.

In einer weiteren Ausführungsform ist vorgesehen, für die Erstellung des Kalibriermodells spektrale Daten aus dem NIR- Spektralbereich zwischen 1450 und 1550 nm zu verwenden, die mittels geeigneter mathematischer Methoden vorbehandelt werden und anschließend der multivariaten Datenanalyse zugeführt werden.

Die Bedeutung einer Wellenlänge für die Vorhersage des Harzverhältnisses aus dem NIR-Spektrum wird mit Hilfe der Regressionskoeffizienten dargestellt. Dabei haben die Regionen mit großen Koeffizientenbeträgen starken Einfluss auf das Regressionsmodell. So zeigt die Darstellung der Regressionskoeffizienten in einem PLS-Regressionsmodell für die Bestimmung der Harzmenge bzw. des Harzgehaltes, dass der Wellenlängenbereich zwischen 1460 nm und 1530 mit Maximum bei 1490 nm (Absorptionsbande der Amino-Gruppen des Harzes) für die Berechnung des Modells am wichtigsten ist, da hier die Beträge der Regressionskoeffizienten am größten sind. Die anderen Bereiche im Spektrum haben zwar geringeren Informationsgehalt in Bezug auf die NIR-Messung, tragen aber dennoch dazu bei, die weiteren Informationen bzw. störenden Einflussgrößen (wie Transparenz der Schicht, Oberflächenbeschaffenheit des Trägermaterials usw.) zu berücksichtigen bzw. zu minimieren.

Zur Eliminierung von störenden Einflüssen (wie z.B. Beschaffenheit der Oberfläche des Trägermaterials, Farbigkeit der Proben, Lichtstreuung an Feststoffpartikeln oder anderen Additiven usw.) ist es notwendig, die spektralen Daten mit mathematischen Vorbehandlungsmethoden (z. B. derivative Datenvorbehandlung, Standardisierung gemäß SNVT (Standard Normal Variate Transformation), multiplikative Signal-Korrektur (EMSC, Extended Multiplicative Signal Correction usw.) zu bearbeiten. Dabei werden die Basislinieneffekte, die hauptsächlich durch die unterschiedliche Farbe der Proben verursacht werden, aus den Spektren entfernt, überlagernde Banden voneinander getrennt und die Abhängigkeit der Lichtstreuung an der Substratoberfläche oder an den Feststoffpartikeln in der Beschichtung berücksichtigt. Bei der Vermessung einer Dekorpapierlage liegt der Schwerpunkt der Kalibrierung und Datenvorbehandlung auf der Entfernung der Basislinienverschiebung.

Aus den vorbehandelten Daten wird mit Hilfe der multivariaten Datenanalyse ein Kalibriermodell entwickelt, das alle bei der Kalibrierung verwendeten Dekore beinhaltet.

Entsprechend erfolgen der Vergleich und die Interpretation der NIR-Spektren bevorzugt im Spektralbereich zwischen 1450 und 1550 nm unter Verwendung der multivariaten Datenanalyse MDA. Bei multivariaten Analysemethoden werden in an sich bekannter Weise typischerweise mehrere statistische Variablen zugleich untersucht. Hierzu wird die in einem Datensatz enthaltene Zahl von Variablen reduziert, ohne gleichzeitig die darin enthaltene Information zu mindern.

Wie oben beschrieben, wird vorliegend das Verhältnis von Melamin-Formaldehyd-Harz und Harnstoff-Formaldehyd-Harz in einer (vor)imprägnierten Papierlage bestimmt. Als Papierlage kommen z.B. Overlaypapiere, Dekorpapiere, oder Kraftpapiere zum Einsatz. Overlaypapiere sind dünne Papiere, welche typischerweise bereits mit einem konventionellen Melaminharz getränkt wurden Es sind ebenfalls Overlaypapiere erhältlich, in denen abriebfeste Partikel, wie zum Beispiel Korundpartikel in das Harz des Overlays eingemischt sind oder auf das imprägnierte Overlay aufgestreut werden, um die Abriebfestigkeit zu erhöhen. Dekorpapiere sind Spezialpapiere zur Oberflächenveredelung von Holzwerkstoffen, die eine hohe Dekorvielfalt ermöglichen. So sind neben den typischen Aufdrucken von diversen Holzstrukturen weitergehende Aufdrucke von geometrischen Formen oder künstlerischen, dekorativen Ausarbeitungen erhältlich. Eine Einschränkung in der Motivwahl gibt es faktisch nicht. Um eine optimale Bedruckbarkeit zu gewährleisten muss das verwendete Papier eine gute Glätte und Dimensionsstabilität aufweisen und ebenfalls für eine Penetration einer notwendigen Kunstharzimprägnierung geeignet sein. Kraftpapiere weisen eine hohe Festigkeit auf und bestehen aus Zellstofffasern, denen Stärke, Alaun und Leim zugesetzt sind, um Festigkeitssteigerungen zu erzielen.

In einer bevorzugten Ausführungsform ist die Papierlage teilweise oder vollständig mit dem Harz imprägniert, wobei das Harz in das Trägermaterial eindringt bzw. hinein penetriert. Vorliegend ist unter dem Begriff der "Imprägnierung" eine vollständige oder teilweise Durchtränkung der Papierlage mit dem Harz zu verstehen. Insbesondere wird das vorliegende Verfahren bei vor- bzw. kernimprägnierten Papierlagen verwendet.

Das vorliegende Verfahren zur Bestimmung des quantitativen Verhältnisses von Melamin- und Harnstoff-Formaldehyd-Harz in einer vorimprägnierten Papierlage kann kontinuierlich und online in einer Imprägnieranlage erfolgen.

Eine entsprechende Imprägnieranlage zur Imprägnierung von mindestens einer durch die Anlage laufenden Papierlage umfasst dabei:
- optional Papierabwicklung und Wechselvorrichtung,
- eine erste Imprägnierstation zur Vorimprägnierung (Kernimprägnierung) von mindestens einer Papierlage mit einer Harzmischung aus Melamin-Formaldehyd-Harz und Harnstoff-Formaldehyd-Harz, und
- eine zweite Imprägnierstation zur Oberflächenimprägnierung der mindestens einen vorimprägnierten Papierlage,
- wobei zwischen der ersten Imprägnierstation und der zweiten Imprägnierstation mindestens ein NIR-Messkopf, insbesondere mindestens ein NIR-Multimesskopf, zur Aufnahme von mindestens einem NIR-Spektrum der imprägnierten Papierlage nach der Vorimprägnierung in der ersten Imprägnierstation vorgesehen ist,
- optional Clipper und Ablegetisch

Der NIR-Messkopf ist bevorzugt so angeordnet, dass die Oberseite der imprägnierten Papierlage bestrahlt wird. Generell ist es aber auch möglich weitere NIR-Messköpfe in der Imprägnieranlage zur Vermessung der Oberseite und Unterseite der imprägnierten Papierlage vorzusehen.

Es ist insbesondere bevorzugt, wenn der mindestens eine NIR-Messkopf über die Breite der Papierlage traversiert und bestimmte Problembereiche (z. B. im Rand- oder Mittelbereich der Papierlage usw.) analysiert. Entsprechend bewegt sich der mindestens eine NIR-Messkopf quer zur Laufrichtung der (vor)imprägnierten Papierlage. Außerdem stehen die Messwerte sofort zur Verfügung und erlauben einen sofortigen Eingriff in den Prozess. Dies ist bei anderen Verfahren nicht ohne weiteres möglich.

In einer Ausführungsform der vorliegenden Imprägnieranlage ist nach der ersten Imprägnierstation eine erste Trockenstation vorgesehen, wobei der mindestens eine NIR-Messkopf nach der ersten Trockenstation angeordnet ist.

In einer konkreteren Ausführungsform umfasst die Imprägnieranlage Imprägnierwannen bzw. Imprägniertauchbäder (als Imprägnierstationen), ggfs. eine Atemstrecke, ein Rakelsystem / Quetschwalzenpaar zur Entfernung von überschüssigem Harz, optional eine Vorrichtung zum Streuen von abriebfesten Partikeln, mindestens einen Trockner (z.B. ein Schwebetrockner), optional ein Rasterwerk und ein optional zweiter Trockner, mindestens eine Abkühlvorrichtung (z.B. ein Kühlwalzsystem).

Es wird somit ein Verfahren bereitgestellt, in welchem durch die Verwendung eines NIR-Messkopfes, das quantitative Verhältnis von Melamin-Formaldehyd-Harz und Harnstoff-Formaldehyd-Harz in einer (vor)imprägnierten Papierlage aus einem NIR-Spektrum bestimmt werden kann, und zwar durch eine berührungslose Messung. Die mit dem Messkopf oder den Messköpfen ermittelten Daten werden direkt zur Anlagensteuerung oder -regelung verwendet.

Zudem wird in einer weiteren vorteilhaften Ausprägung der Erfindung durch die Speicherung der Daten eine Verbesserung der Qualitätskontrolle ermöglicht. Auch bei Anlagenversuchen können die gespeicherten Daten vorteilhaft einen Beitrag zur Auswertung liefern, z.B. Inbetriebnahme einer Anlage bei Neuinstallation oder nach einer Wartung oder Reparatur oder zu in-situ Testzwecken von neuen Produktions- oder Messverfahren. Durch das sofortige Vorliegen der Messwerte und der hohen Messfrequenz wird eine sehr enge Kontrolle bzw. Steuerung oder Regelung der Anlagen ermöglicht.

Das vorliegende Verfahren ermöglicht die Bereitstellung der Messwerte in kurzer Zeit (online, bevorzugt ohne störende Zeitverzögerung) im Vergleich zu herkömmlichen (bekannten) Messverfahren. Die Messdaten können zur Qualitätssicherung, Forschung und Entwicklung, zur Prozesskontrolle, Prozessregelung, Prozesssteuerung usw. eingesetzt werden. Durch den Messvorgang wird die Produktionsgeschwindigkeit usw. nicht reduziert. Grundsätzlich wird damit die Überwachung der Produktion verbessert. Zudem werden auch Stillstandszeiten durch Qualitätsbestimmungen und Anlagenjustierungen reduziert.

Die Vorteile des vorliegenden Verfahrens sind vielfältig: Berührungslose Multiparameterbestimmung ("real time"- oder "Echtzeit"-Messung) mit deutlich reduzierter zeitlicher Verzögerung in der Auswertung der gemessenen Parameterwerte; verbesserte Anlagensteuerung bzw. -regelung, Verringerung des Ausschusses, Verbesserung der Qualität der auf der Anlage hergestellten Produkte, Verbesserung der Anlagenverfügbarkeit.

Das Steuerungssystem der Imprägnieranlage umfasst mindestens eine computergestützte Auswerteeinheit (bzw. Prozessoreinheit) und eine Datenbank. In der Auswerteeinheit erfolgt der Abgleich bzw. Vergleich des für das Produkt (d.h. verpresstes poröses Beschichtungsmaterial) gemessenen NIR-Spektrums mit den für die jeweils einzelnen Parameter erstellten Kalibriermodellen. Die so bestimmten Parameterdaten werden in der Datenbank gespeichert.

Die mit dem vorliegenden spektroskopischen Verfahren bestimmten Daten werden zur Steuerung der Imprägnieranlage verwendet. Die berührungslos gemessenen Parameterwerte des NIR-Multimesskopfes ("Ist-Werte") können, wie zuvor bereits beschrieben, direkt und in "real time" für die Steuerung bzw. Regelung der betreffenden Anlage verwendet werden, indem beispielsweise die gemessenen und in der Datenbank, z.B. einer relationalen Datenbank, Ist-Werte gespeichert und mit dort vorhandenen Soll-Werten dieser Parameter verglichen werden. Die sich ergebenden Differenzen werden anschließend zur Steuerung bzw. Regelung der Produktionslinie verwendet.

Für den Abgleich und die Steuerung der Imprägnieranlage wird ein computerimplementiertes Verfahren sowie ein Computerprogramm umfassend Befehle, die bei der Ausführung des Programms durch einen Computer diesen veranlassen das computerimplementierte Verfahren auszuführen, bereitgestellt. Das Computerprogramm ist in einer Speichereinheit des Steuerungssystems der Imprägnieranlage gespeichert.

Die Erfindung wird nachfolgend an Ausführungsbeispielen näher erläutert.

### Herstellung der imprägnierten Papiere

Zur Herstellung der imprägnierten Papiere wird das Dekorpapier (Papiergewicht: 65 g/m², 30 x 30 cm) mit den folgenden 3 Mischungsverhältnissen von Harnstoffharz (HF, Metadynea Primere 70 1093L, Festharzgehalt: 52 Gew%) und Melaminharz (MF, Metadynea Primere 700867L, Festharzgehalt: 62 Gew%) imprägniert (s. Tabelle 1).

**Tabelle 1: 3 Mischungsverhältnisse zur Harzmischung von MF(Gew%) und HF(Gew%)**

| Variante | Harzmischung MF und HF ( in g) | MF ( in g fl.) | MF im Harz (in g fest) | HF ( in g fl.) | HF ( in g fest) | MF (Gew%) / HF (Gew%) |
|---|---|---|---|---|---|---|
| 1 | 10 | 10 | 6,2 | 0 | 0 | 100*: 0 |
| 2 | 10 | 8 | 4,96 | 2 | 1,04 | 83*: 17 |
| 3 | 10 | 2 | 1,24 | 8 | 4,16 | 25*: 75 |

| | | | | | | |
|---|---|---|---|---|---|---|
| *MF Gew% wird als Referenzwert zum Erstellen des Kalibrationsmodells verwendet | | | | | | |

Bei jeder Imprägnierung wird 10 g Harzmischung auf das Dekorpapier aufgetragen. Zur Beschleunigung der Aushärtung des Harzes wird 0,1 g Härter (Alton HM 1448) zur Harzmischung zugegeben. Die imprägnierten Papiere werden im Trockenschrank auf ca. 6 Gew% getrocknet.

### Erstellung des Kalibrationsmodells

Das imprägnierte Papier als Referenzprobe wird mittels NIR vermessen. Um ein gutes statistisches Kalibrationsmodell zu erstellen, werden bei der Aufnahme der NIR-Spektren mehrere Messpunkte ( ca. 15 ) auf die Probe gemessen. Bei jedem Messpunkt werden 2 Messungen wiederholt. Die Aufnahme der NIR-Spektren im Labor erfolgte mit dem Messsystem DA 7250 (Perten Instruments GmbH).

Nach der Aufnahme der NIR-Spektren wird MF in Gew% in Korrelation mit spektralen Daten gebracht. Die Erstellung des Kalibrationsmodells erfolgt mittels multivariater Datenanalyse. Dies geschieht mit einer geeigneten Analysesoftware zum Beispiel mit "The Unscrambler" der Firma CAMO.

Aus den Referenzspektren wird ein Kalibrationsmodell erstellt, welches zu Ermittlung (Vorhersage) der quantitativen Prüfung des Harnstoffharzes einer unbekannten Probe genutzt werden kann.

Insgesamt werden 15 NIR-Spektren zum Erstellen des Kalibrationsmodells verwendet. Es werden 7 NIR-Spektren der Variante 1 aufgenommen. Von der Variante 2 und der Variante 3 werden jeweils 4 NIR-Spektren aufgenommen. Alle 15 NIR-Spektren werden mit Datenvorbehandlungen Detrend durchgeführt. Anschließend wird das PLS-Modell erstellt. Die Parameter des berechneten Modells werden in Tabelle 2 zusammenfassend dargestellt.

**Tabelle 2: PLS-Modell zur quantitativen Bestimmung von Harnstoffharz in melaminharzimprägnierten Papieren**

| Modell | Datenvorbehandlung | PLS | RMSECV | R² | Bias | Slope | Offset |
|---|---|---|---|---|---|---|---|
| 1 | Detrend | 3^{*} | 3,94 | 0,98 | -0,33 | 0,98 | 0,59 |

| | | | | | | | |
|---|---|---|---|---|---|---|---|
| *vom Programmen The Unscrambler vorgeschlagene optimale Anzahl der Hauptfaktoren | | | | | | | |

### Validierung des Kalibrationsmodells

Zum Testen des obengenannten Kalibrationsmodells wird das Imprägnat der Varianten 2 und 3, die in Tabelle 1 beschrieben werden, erneut mit den obigen Harz-Verhältnissen hergestellt. Zusätzlich wird eine 10 g Harzmischung aus 5 g MF und 5 g HF hergestellt. Diese neue Mischung wird als Verhältnis 4 bezeichnet. Das Verhältnis 4 dient als unbekannte Probe zur Prüfung des Kalibrationsmodells. Folgenden Parameter der unbekannten Probe können aus der Tabelle 3 entnommen werden.

**Tabelle 3: Mischungsverhältnis 4 zur Validierung des Kalibrationsmodells**

| Mischungs verhältnis | MF und HF( in g fl.) | Gewicht von MF(g) | Festharzgehalt von MF in der Harzmischung (g) | Gewicht von HF(g) | Festharzgehalt von HF in der Harzmischung (g) | MF (Gew%) / HF (Gew%) |
|---|---|---|---|---|---|---|
| 4 | 10 | 5 | 3,1 | 5 | 2,6 | 54*: 46 |

| | | | | | | |
|---|---|---|---|---|---|---|
| *MF in Gew% wird als Referenzwert zum Testen des Kalibrationsmodells verwendet | | | | | | |

Mit dem Mischungsverhältnis 2, 3 und 4 werden jeweilige 2 Proben mittels oben beschriebenen Herstellungsprozessen produziert, um das Kalibrationsmodell zu testen. Aus jeder Probe wird ein Messwert des MF Gew% mittels Kalibrationsmodell ermittelt. Insgesamt ergeben sich 6 Messwerte von 6 Proben (s. Tabelle 4). Der MF-Mittelwert von beiden Probenmessungen wird ermittelt.

**Tabelle 4: Zusammenfassung der Validierung des ersten Kalibrationsmodells.**

| Variante | Referenzwert von MF(Gew%) | Messwert von MF der Probe 1 (Gew%) | Messwert von MF der Probe 2 (Gew%) | MF-Mittelwert von Proben 1 und 2 (Gew%) | Abweichung zwischen Referenzwert und Mittelwert (Gew%) |
|---|---|---|---|---|---|
| 2 | 83 | 70 | 72 | 71 | -12 |
| 3 | 25 | 14 | 16 | 15 | -10 |
| 4 | 54 | 48 | 50 | 49 | -5 |

Zwar ist Abweichung zwischen dem Referenzwert und dem Mittelwert der Messungen vom Mischungsverhältnis 2 und 3 größer als 10 Gew%, jedoch ist bei dem Mischungsverhältnis 4 die Abweichung zwischen Referenzwert und Mittelwert der Messungen 5 Gew% deutlich kleiner als bei dem Verhältnis 2 und 3.

Aus der praktischen Erfahrung wird eine Harzmischung zwischen MF und HF von bis zu 50 %: 50% bei imprägnierten Papier bei der Vorimprägnierung verwendet. Diese Ergebnisse der Tabelle 4 zeigen, wenn das Harzmischungsverhältnis von MF und HF bei 50 Gew% liegt, kann das Kalibrationsmodell die quantitativen Mengen von Harnstoff in melaminimprägniertes Papier genau bestimmen.

Die on-line Messung der Harzverhältnisse erfolgt nach dem ersten Trockner, da dort die Wassermenge auf ca. 10 bis 20 Gew% reduziert ist und nicht mehr den Harnstoffpeak überlagert. Der Messkopf sollte über die Papierbahn traversieren, um auch Unterschiede über die Breite zu erfassen.

## Patentansprüche

1. Verfahren zur Bestimmung des quantitativen Verhältnisses von Melamin-Formaldehyd-Harz und Harnstoff-Formaldehyd-Harz in mindestens einer mit einer Mischung aus diesen Harzen imprägnierten Papierlage, wobei die Imprägnierung in einer Anlage zur Imprägnierung von mindestens einer durch die Anlage laufenden Papierlage durchgeführt wird,
umfassend die Schritte
- Imprägnieren von mehreren Papierlagen mit Harzmischungen mit jeweils unterschiedlichen quantitativ definierten Verhältnissen an Melamin-Formaldehyd-Harz und Harnstoff-Formaldehyd-Harz als Referenzproben;
- Aufnahme von mindestens einem NIR-Spektrum jeder der Referenzproben unter Verwendung von mindestens einem NIR-Messkopf in einem Wellenlängenbereich zwischen 500 nm und 2500 nm, bevorzugt zwischen 700 nm und 2000 nm, insbesondere bevorzugt zwischen 900 nm und 1700 nm, und besonders vorteilhaft zwischen 1450 nm und 1550 nm;
- Zuordnung der unterschiedlichen quantitativen Verhältnisse an Melamin-Formaldehyd-Harz und Harnstoff-Formaldehyd-Harz in den Referenzproben zu den aufgenommenen NIR-Spektren der genannten Referenzproben; und
- Erstellung eines Kalibriermodells für den Zusammenhang zwischen den spektralen Daten der NIR-Spektren und den dazugehörigen quantitativen Verhältnissen an Melamin-Formaldehyd-Harz und Harnstoff-Formaldehyd-Harz der Referenzproben mittels einer multivariaten Datenanalyse;
- Imprägnieren von mindestens einer Papierlage mit einer Harzmischung aus Melamin-Formaldehyd-Harz und Harnstoff-Formaldehyd-Harz;
- Aufnehmen von mindestens einem NIR-Spektrum der imprägnierten Papierlage unter Verwendung des mindestens einen NIR-Messkopfes in einem Wellenlängenbereich zwischen 500 nm und 2500 nm, bevorzugt zwischen 700 nm und 2000 nm, insbesondere bevorzugt zwischen 900 nm und 1700 nm und besonders vorteilhaft zwischen 1450 nm und 1550 nm; und
- Bestimmen des quantitativen Verhältnisses von Melamin-Formaldehyd-Harz und Harnstoff-Formaldehyd-Harz in der imprägnierten Papierlage durch Vergleich des für die imprägnierte Papierlage aufgenommenen NIR-Spektrums mit dem erstellten Kalibriermodell,
wobei die unter Einsatz des mindestens einen Messkopf ermittelten Daten zur Anlagensteuerung und Anlagenregelung verwendet werden.

2. Verfahren nach Anspruch 1, **dadurch gekennzeichnet, dass** die Bestimmung des quantitativen Verhältnisses von Melamin-Formaldehyd-Harz und Harnstoff-Formaldehyd-Harz in einer vorgetrockneten imprägnierten Papierlage erfolgt.

3. Verfahren nach einem der vorhergehenden Ansprüche, **dadurch gekennzeichnet, dass** die Bestimmung des quantitativen Verhältnisses von Melamin-Formaldehyd-Harz und Harnstoff-Formaldehyd-Harz in mindestens einer mit dieser Harzmischung vorimprägnierten (bzw. kernimprägnierten) Papierlage erfolgt.

4. Verfahren nach einem der vorhergehenden Ansprüche, **dadurch gekennzeichnet, dass** die Bestimmung des quantitativen Verhältnisses von Melamin-Formaldehyd-Harz und Harnstoff-Formaldehyd-Harz in mindestens einer mit dieser Harzmischung vorimprägnierten (bzw. kernimprägnierten) Papierlage nach Verlassen einer (ersten) Imprägnierstation als Teil einer Anlage zur Imprägnierung von mindestens einer durch die Anlage laufenden Papierlage vorgenommen wird.

5. Verfahren nach Anspruch 4, **dadurch gekennzeichnet, dass** im Anschluss an Vorimprägnierung der Papierlage in einer ersten Imprägnierstation eine Oberflächenimprägnierung der vorimprägnierten Papierlage in einer zweiten Imprägnierstation erfolgt.

6. Verfahren nach einem der vorhergehenden Ansprüche, **dadurch gekennzeichnet, dass** die Bestimmung des quantitativen Verhältnisses von Melamin-Formaldehyd-Harz und Harnstoff-Formaldehyd-Harz in einer vorimprägnierten Papierlage kontinuierlich und online erfolgt.

7. Verfahren nach einem der vorhergehenden Ansprüche, **dadurch gekennzeichnet, dass** das quantitative Verhältnis von Melamin-Formaldehyd-Harz und Harnstoff-Formaldehyd-Harz in der zur Vorimprägnierung verwendeten Harzmischung zwischen 90 Gew% : 10 Gew% und 10 Gew% : 90 Gew%, bevorzugt zwischen 75 Gew% : 25 Gew% und 25 Gew%: 75 Gew%, insbesondere bevorzugt zwischen 55 Gew%: 45 Gew% und 45 Gew%: 55 Gew% beträgt.

8. Verfahren nach einem der vorhergehenden Ansprüche, **dadurch gekennzeichnet, dass** für die Erstellung des Kalibriermodells spektrale Daten aus dem gesamten aufgenommenen Spektralbereich verwendet werden.

9. Verfahren nach einem der vorhergehenden Ansprüche, **dadurch gekennzeichnet, dass** für die Erstellung des Kalibriermodells spektrale Daten aus dem NIR-Spektralbereich zwischen 1450 nm und 1550 nm verwendet werden, die mittels geeigneter mathematischer Methoden vorbehandelt werden und anschließend der multivariaten Datenanalyse zugeführt werden.

10. Verfahren nach einem der vorhergehenden Ansprüche, **dadurch gekennzeichnet, dass** der mindestens eine NIR-Messkopf über die Breite der Papierlage traversiert und Problembereiche, insbesondere im Rand- oder Mittelbereich der Papierlage, analysiert, und die Messwerte zur Anlagenregelung und Anlagensteuerung verwendet werden.

11. Verfahren nach einem der vorhergehenden Ansprüche, **dadurch gekennzeichnet, dass** der Vergleich der Daten des gemessenen NIR-Spektrums mit den erstellten Kalibriermodellen in einer computergestützten Auswerteeinheit des Steuerungssystems erfolgt und die so bestimmten Parameterdaten werden in einer Datenbank des Steuerungssystems gespeichert werden.

12. Verfahren nach Anspruch 11, **dadurch gekennzeichnet, dass** die berührungslos gemessenen Parameterwerte des mindestens einen NIR-Messkopfes als Ist-Werte in der Datenbank gespeichert werden und mit in der Datenbank vorhandenen Soll-Werten dieser Parameter verglichen werden, wobei die sich ergebenden Differenzen zur Steuerung bzw. Regelung der Anlage verwendet werden.

13. Verfahren nach Anspruch 11, **dadurch gekennzeichnet, dass** die gespeicherten Daten zur Qualitätskontrolle verwendet werden.

14. Verfahren nach Anspruch 11, **dadurch gekennzeichnet, dass** die gespeicherten Daten bei der Inbetriebnahme einer Anlage bei Neuinstallation oder nach einer Wartung oder Reparatur oder zu in-situ Testzwecken von neuen Produktions- oder Messverfahren verwendet werden.

## Claims

1. A method of determining the quantitative ratio of melamine-formaldehyde resin and urea-formaldehyde resin in at least one paper layer impregnated with a mixture of these resins, the impregnation being carried out in a plant for impregnating at least one paper layer passing through the plant,
comprising the steps of
- Impregnating several paper layers with resin mixtures each having different quantitatively defined ratios of melamine-formaldehyde resin and urea-formaldehyde resin as reference samples;
- Recording of at least one NIR spectrum of each of the reference samples using at least one NIR measuring head in a wavelength range between 500 nm and 2500 nm, preferably between 700 nm and 2000 nm, more preferably between 900 nm and 1700 nm, and particularly advantageously between 1450 nm and 1550 nm;
- Correlating the different quantitative ratios of melamine-formaldehyde resin and urea-formaldehyde resin in the reference samples to the recorded NIR spectra of said reference samples; and
- Establishing a calibration model for the relationship between the spectral data of the NIR spectra and the corresponding quantitative ratios of melamine-formaldehyde resin and urea-formaldehyde resin of the reference samples by means of multivariate data analysis;
- Impregnating at least one paper layer with a resin mixture of melamine-formaldehyde resin and urea-formaldehyde resin;
- Recording at least one NIR spectrum of the impregnated paper layer using the at least one NIR measuring head in a wavelength range between 500 nm and 2500 nm, preferably between 700 nm and 2000 nm, more preferably between 900 nm and 1700 nm and particularly advantageously between 1450 nm and 1550 nm; and
- Determining the quantitative ratio of melamine-formaldehyde resin and urea-formaldehyde resin in the impregnated paper layer by comparing the **NIR** spectrum recorded for the impregnated paper layer with the calibration model created,
wherein the data determined using the at least one measuring head are used for plant control and plant regulation.

2. Method according to claim 1, **characterized in that** the determination of the quantitative ratio of melamine-formaldehyde resin and urea-formaldehyde resin is carried out in a pre-dried impregnated paper layer.

3. Method according to one of the preceding claims, **characterized in that** the determination of the quantitative ratio of melamine-formaldehyde resin and urea-formaldehyde resin is carried out in at least one paper layer pre-impregnated (or core-impregnated) with this resin mixture.

4. Method according to one of the preceding claims, **characterized in that** the determination of the quantitative ratio of melamine-formaldehyde resin and urea-formaldehyde resin in at least one paper layer pre-impregnated (or core-impregnated) with this resin mixture is carried out after leaving a (first) impregnation station as part of a plant for impregnating at least one paper layer passing through said plant.

5. Method according to claim 4, **characterized in that**, following pre-impregnation of the paper layer in a first impregnation station, surface impregnation of the pre-impregnated paper layer takes place in a second impregnation station.

6. Method according to one of the preceding claims, **characterized in that** the determination of the quantitative ratio of melamine-formaldehyde resin and urea-formaldehyde resin in a pre-impregnated paper layer is carried out continuously and online.

7. Method according to one of the preceding claims, **characterized in that** the quantitative ratio of melamine-formaldehyde resin and urea-formaldehyde resin in the resin mixture used for pre-impregnation is between 90 wt% : 10 wt% and 10 wt% : 90 wt%, preferably between 75 wt% : 25 wt% and 25 wt% : 75 wt%, more preferably between 55 wt% : 45 wt% and 45 wt% : 55 wt%.

8. Method according to one of the preceding claims, **characterized in that** spectral data from the entire recorded spectral range are used to create the calibration model.

9. Method according to one of the preceding claims, **characterized in that** spectral data from the NIR spectral range between 1450 nm and 1550 nm are used to create the calibration model, which are pretreated by means of suitable mathematical methods and then fed to the multivariate data analysis.

10. Method according to one of the preceding claims, **characterized in that** the at least one NIR measuring head traverses across the width of the paper layer and analyses problem areas, in particular in the edge or middle region of the paper layer, and the measured values are used for plant regulation and plant control.

11. Method according to one of the preceding claims, **characterized in that** the comparison of the data of the measured NIR spectrum with the calibration models created is carried out in a computer-aided evaluation unit of the control system and the parameter data determined in this way are stored in a database of the control system.

12. Method according to claim 11, **characterized in that** the non-contact measured parameter values of the at least one NIR measuring head are stored as actual values in the database and are compared with target values of these parameters present in the database, the resulting differences being used to control or regulate the plant.

13. Method according to claim 11, **characterized in that** the stored data are used for quality control.

14. Method according to claim 11, **characterized in that** the stored data are used during the commissioning of a plant during a new installation or after maintenance or repair or for in-situ testing purposes of new production or measuring processes.

## Revendications

1. Procédé de détermination du rapport quantitatif entre la résine mélamine-formaldéhyde et la résine urée-formaldéhyde dans au moins une couche de papier imprégnée d'un mélange desdites résines, dans lequel l'imprégnation est effectuée dans une installation d'imprégnation d'au moins une couche de papier traversant l'installation,
comprenant les étapes
- d'imprégnation de plusieurs couches de papier avec des mélanges de résines, avec respectivement des rapports quantitativement définis différents de résine mélamine-formaldéhyde et de résine urée-formaldéhyde, comme échantillons de référence ;
- d'enregistrement d'au moins un spectre NIR de chacun des échantillons de référence en utilisant au moins une tête de mesure NIR dans une plage de longueurs d'onde entre 500 nm et 2500 nm, de manière préférée entre 700 nm et 2000 nm, en particulier de manière préférée entre 900 nm et 1700 nm, et de manière particulièrement avantageuse entre 1450 nm et 1550 nm ;
- d'association des différents rapports quantitatifs de résine mélamine-formaldéhyde et de résine urée-formaldéhyde dans les échantillons de référence aux spectres NIR enregistrés desdits échantillons de référence ; et
- de création d'un modèle d'étalonnage pour la corrélation entre les données spectrales des spectres NIR et les rapports quantitatifs associés de résine mélamine-formaldéhyde et de résine urée-formaldéhyde des échantillons de référence au moyen d'une analyse de données multivariée ;
- d'imprégnation d'au moins une couche de papier avec un mélange de résine mélamine-formaldéhyde et de résine urée-formaldéhyde ;
- d'enregistrement d'au moins un spectre NIR de la couche de papier imprégnée en utilisant l'au moins une tête de mesure NIR dans une plage de longueurs d'onde entre 500 nm et 2500 nm, de manière préférée entre 700 nm et 2000 nm, en particulier de manière préférée entre 900 nm et 1700 nm, et de manière particulièrement avantageuse entre 1450 nm et 1550 nm ; et
- de détermination du rapport quantitatif de résine mélamine-formaldéhyde et de résine urée-formaldéhyde dans la couche de papier imprégnée en comparant le spectre NIR enregistré pour la couche de papier imprégnée au modèle d'étalonnage créé,
dans lequel les données obtenues à l'aide de l'au moins une tête de mesure sont utilisées pour la commande et la régulation de l'installation.

2. Procédé selon la revendication 1, **caractérisé en ce que** la détermination du rapport quantitatif de résine mélamine-formaldéhyde et de résine urée-formaldéhyde est effectuée dans une couche de papier imprégné préalablement séchée.

3. Procédé selon l'une quelconque des revendications précédentes, **caractérisé en ce que** la détermination du rapport quantitatif de résine mélamine-formaldéhyde et de résine urée-formaldéhyde est effectuée dans au moins une couche de papier préimprégnée (ou imprégnée au centre) avec ledit mélange de résine.

4. Procédé selon l'une quelconque des revendications précédentes, **caractérisé en ce que** la détermination du rapport quantitatif de résine mélamine-formaldéhyde et de résine urée-formaldéhyde dans au moins une couche de papier préimprégnée (ou imprégnée au centre) avec ledit mélange de résine est réalisée après avoir quitté une (première) station d'imprégnation en tant que partie d'une installation d'imprégnation d'au moins une couche de papier traversant l'installation.

5. Procédé selon la revendication 4, **caractérisé en ce qu'**est effectuée, directement après une préimprégnation de la couche de papier dans une première station d'imprégnation, une imprégnation de surface de la couche de papier préimprégnée dans une deuxième station d'imprégnation.

6. Procédé selon l'une quelconque des revendications précédentes, **caractérisé en ce que** la détermination du rapport quantitatif de résine mélamine-formaldéhyde et de résine urée-formaldéhyde dans une couche de papier préimprégnée est effectuée de manière continue et en ligne.

7. Procédé selon l'une quelconque des revendications précédentes, **caractérisé en ce que** le rapport quantitatif de résine mélamine-formaldéhyde et de résine urée-formaldéhyde dans le mélange de résine utilisé pour la préimprégnation est compris entre 90 % en poids: 10 % en poids et 10 % en poids: 90 % en poids, de manière préférée entre 75 % en poids: 25 % en poids et 25 % en poids: 75 % en poids, en particulier de manière préférée entre 55 % en poids: 45 % en poids et 45 % en poids: 55 % en poids.

8. Procédé selon l'une quelconque des revendications précédentes, **caractérisé en ce que** des données spectrales issues de l'ensemble du domaine spectral enregistré sont utilisées pour la création du modèle d'étalonnage.

9. Procédé selon l'une quelconque des revendications précédentes, **caractérisé en ce que** des données spectrales issues du domaine spectral NIR compris entre 1450 nm et 1550 nm sont utilisées pour la création du modèle d'étalonnage, qui sont prétraitées au moyen de méthodes mathématiques appropriées et sont ensuite transmises à l'analyse de données multivariée.

10. Procédé selon l'une quelconque des revendications précédentes, **caractérisé en ce que** l'au moins une tête de mesure NIR traverse la largeur de la couche de papier et analyse des zones problématiques, en particulier dans la zone de bord ou centrale de la couche de papier, et les valeurs mesurées sont utilisées pour la régulation et la commande de l'installation.

11. Procédé selon l'une quelconque des revendications précédentes, **caractérisé en ce que** la comparaison des données du spectre NIR mesuré aux modèles d'étalonnage créés est effectuée dans une unité d'évaluation assistée par ordinateur du système de commande et les données de paramètre ainsi déterminées sont stockées dans une base de données du système de commande.

12. Procédé selon la revendication 11, **caractérisé en ce que** les valeurs de paramètres mesurées sans contact de l'au moins une tête de mesure NIR sont stockées comme des valeurs réelles dans la base de données et sont comparées à des valeurs de consigne desdits paramètres existantes dans la base de données, dans lequel les différences résultantes sont utilisées pour commander ou réguler l'installation.

13. Procédé selon la revendication 11, **caractérisé en ce que** les données stockées sont utilisées pour le contrôle de la qualité.

14. Procédé selon la revendication 11, **caractérisé en ce que** les données stockées sont utilisées lors de la mise en service d'une installation, d'une nouvelle installation ou après une maintenance ou une réparation, ou à des fins de test in situ de nouveaux procédés de production ou de mesure.
